# EUROPÄISCHE PATENTANMELDUNG

(11) **EP 0 985 377 A1**
(43) Veröffentlichungstag der Anmeldung: **15.03.2000**
(21) Anmeldenummer: 99114941.0
(22) Anmeldetag: 30.07.1999
(51) Int. Cl.: A61B 6/00

(54) **Kontrollstab für ein bildgebendes Verfahren**

(30) Priorität: 10.09.1998 DE 29816295 U
(71) Anmelder: orto MAQUET GmbH & Co.KG, 76437 Rastatt (DE)
(72) Erfinder: Dr. Pokrandt, Peter, D-76474 Au am Rhein (DE)
(74) Vertreter: Schaumburg, Thoenes & Thurn

(57) **Zusammenfassung**

Ein Kontrollkörper (10,20) für ein bildgebendes Verfahren, bei dem ein Bild eines Objektes aufgrund unterschiedlicher physikalischer Eigenschaften von Teilbereichen erzeugt wird, umfaßt ein Material (14,24) vorgegebener Dichte, das bezüglich der Symmetrieebenen (A-A',B-B') des von dem Kontrollkörper (10,20) umschlossenen Raumes asymmetrisch ausgebildet und/oder angeordnet ist.

## Beschreibung

Die Erfindung betrifft einen Kontrollkörper für ein bildgebendes Verfahren, bei dem ein Bild eines Objektes aufgrund unterschiedlicher physikalischer Eigenschaften von Teilbereichen erzeugt wird.

Zum Beispiel die Computertomographie (CT), ein Schichtaufnahmeverfahren, wird u.a. bei der Fertigung von Prothesen angewendet. Dabei wird aufgrund der Abschwächung von Röntgenstrahlung eine Verteilung der verschiedenen Gewebebereiche in einer Körperschicht sichtbar gemacht. Ein Computertomograph bestimmt von der zu untersuchenden Körperregion eines Patienten, z.B. des Oberschenkels, die Verteilung von Gewebe in mehreren Körperschichten. Eine Datenverarbeitungsanlage bildet aus den einzelnen Schichtaufnahmen ein drei-dimensionales CT-Bild. Dieses CT-Bild wird anschließend als Vorlage z.B. für die Fertigung einer Prothese benutzt. Als bildgebendes Verfahren kann auch das Magnetresonanzverfahren (MR) verwendet werden.

Um die Brauchbarkeit des CT-Bildes beurteilen zu können, wird neben die aufzunehmende Körperregion ein CT-Kontrollstab gelegt. Dieser CT-Kontrollstab besteht aus einem Rohr, in dem scheibenförmige Abschnitte von Material unterschiedlicher Dichte angeordnet sind. Das Rohr selbst kann aus Kunststoff oder Metall bestehen. Für die Füllung kann ein beliebiges Material verwendet werden, insbesondere Keramik, Glas, Holz oder Kunststoff. Das CT-Bild des CT-Kontrollstabes wird als Vergleichsbild mit unterschiedlichen Schattierungen verwendet. Ferner kann anhand des bekannten Aufbaus des abgebildeten CT-Kontrollstabes festgestellt werden, ob im CT-Bild einzelne Schichtaufnahmen fehlen und ob die aufgenommene Körperregion während des Aufnahmevorgangs relativ zum Tomographen bewegt wurde. Die Verwendung von Materialien bekannter Dichte für die Bestandteile des CT-Kontrollstabes erlaubt eine Kalibrierung der Geräteeinstellung.

Es kann vorkommen, daß aufgrund einer fehlerhaften Datenübertragung oder bei der Bedienung eines Programmes, mit dem das CT-Bild bearbeitet wird, auf einem Datensichtgerät ein gespiegeltes, also seitenverkehrtes Bild dargestellt wird. Statt eines aufgenommenen z.B. rechten Oberschenkelknochens wird dann auf dem Datensichtgerät scheinbar ein linker Oberschenkelknochen dargestellt. Ein CT-Kontrollstab vorstehend genannter Art läßt eine solche seitenverkehrte Darstellung nicht erkennen, da er ein symmetrischer Körper ist.

Aufgabe der Erfindung ist es, für ein bildgebendes Verfahren einen Kontrollkörper zur Verfügung zu stellen, der eine seitenverkehrte Darstellung eines aufgenommenen Objektes erkennbar macht.

Die Erfindung löst die Aufgabe durch einen Kontrollkörper mit den im Anspruch 1 angegebenen Merkmalen. Vorteilhafte Weiterbildungen der Erfindung sind Gegenstand der Unteransprüche.

Die Erfindung beruht auf der Erkenntnis, daß die Abbildung eines symmetrischen Kontrollkörpers, der ein asymmetrisches Merkmal hat, bei verkehrten Seiten sofort als falsch erkannt werden kann, da das asymmetrische Merkmal dann in der Abbildung des Körpers eine Positionsänderung gegenüber seiner ursprünglichen Lage erfährt. Dieses Merkmal des Kontrollkörpers, das bezüglich aller Symmetrieebenen des Kontrollkörpers asymmetrisch ist, liegt außerhalb aller dieser Symmetrieebenen und erfährt daher bei einer Spiegelung eine Positionsänderung gegenüber seiner ursprünglichen Lage.

Der Kontrollkörper selbst kann asymmetrisch ausgebildet sein oder er kann eine bezüglich der Symmetrieebenen des Kontrollkörpers asymmetrisch angeordnete Markierung tragen. Ein Beispiel für einen asymmetrisch ausgebildeten Kontrollkörper ist eine freitragende schraubenförmige Spirale. Ein so ausgebildeter Kontrollkörper umschließt einen symmetrischen Raum, besitzt aber einen Windungssinn, der bei einer seitenverkehrten Darstellung umgekehrt wird.

Eine mögliche Ausführungsform eines Kontrollkörpers mit einer Markierung ist ein Vierkantstab mit quadratischem oder rechteckigem Querschnitt. Als Markierung könnte ein bezüglich der Symmetrieebenen des Vierkantstabes asymmetrisch liegendes Rechteck verwendet werden, das z.B. auf die Mantelfläche des Vierkantstabes aufgebracht wird.

Als weitere Ausführungsform für einen Kontrollkörper ist ein Zylinderstab denkbar. Bei dieser Ausführungsform kann die Markierung die Form eines schräg zur Längsachse des Zylinders verlaufenden Streifens haben. Da ein Zylinder unendlich viele Symmetrieebenen hat, die sich alle in der Längsachse des Zylinders schneiden, schneidet dieser Streifen unendlich viele Symmetrieebenen. Der Streifen ist aber bezüglich jeder dieser geschnittenen Symmetrieebenen asymmetrisch, da er die Symmetrieebenen nicht rechtwinklig schneidet. Der Streifen kann auch als eine den Zylinderstab umgebende Wendel ausgebildet sein.

Um zu vermeiden, daß die Markierung auf die Mantelfläche aufgeklebt werden muß, könnte die Markierung in den Kontrollkörper eingebettet werden. Da ein Klebstoff u.U. eine von den übrigen verwendeten Materialien verschiedene Dichte hat, würde er ebenfalls auf einem Datensichtgerät dargestellt.

Der Kontrollkörper kann aus einem beliebigen Material gefertigt werden. Es eignen sich jedoch besonders Keramik, Glas, Holz und Kunststoff. Kontrollkörper aus diesen Materialien sind auf Grund ihres geringen Gewichts gut zu handhaben.

Für die Markierung kann ein beliebiges Material verwendet werden, dessen Dichte sich von der Dichte der übrigen verwendeten Materialien unterscheidet. Zur Herstellung einer Wendel, die auf der Mantelfläche eines zylinderförmigen Kontrollkörpers aufgebracht werden soll, ist es zweckmäßig z.B. einen Draht oder ein Metallband zu verwenden, mit denen der Kontrollkörper umwickelt werden kann.

Der eingangs genannte CT-Kontrollstab kann, nach dem er wendelförmig mit einem Draht oder einem Metallband umwickelt wurde, zur Erkennung seitenverkehrter Darstellungen benutzt werden. Eine solche Markierung kann auch an der Innenseite des Rohres angebracht werden. Diese Ausführungsform hat den Vorteil, daß die Markierung durch den Rohrmantel geschützt wird und ein Verrutschen vermieden wird.

Nachfolgend wird die Erfindung anhand der Zeichnungen näher erläutert. Darin zeigen:
- Figur 1: einen zylinderförmigen Kontrollkörper,
- Figur 2: die Seitenansicht eines weiteren Kontrollkörpers und
- Figur 3: die Ansicht III ― III des Kontrollkörpers aus Figur 2.

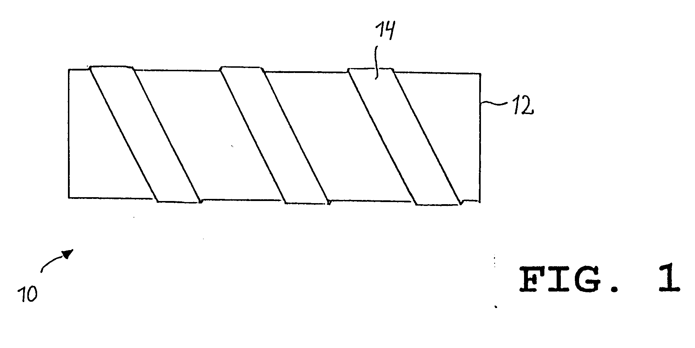

Figur 1 zeigt als Beispiel für die Erfindung einen ersten Kontrollstab 10. Der erste Kontrollstab 10 besteht aus einem Zylinderstab 12 und einer Wendel 14, die um den Zylinderstab 12 gewickelt ist. Die Wendel 14 besitzt einen Windungssinn, der sich bei einer fehlerhaften seitenverkehrten Darstellung umkehrt. Anhand des Windungssinns kann also sogleich festgestellt werden, ob eine ordnungsgemäße Darstellung vorliegt oder nicht. Die Wendel 14 kann auch freitragend ausgeführt sein, wenn sie aus einem ausreichend steifen Material besteht. Sie ist dann symmetrisch bezüglich der Symmetrieebenen des von ihr umschlossenen Raumes, der bei dem in Figur 1 gezeigten Ausführungsbeispiel von dem Zylinderstab 12 eingenommen wird. Figur 1 zeigt den Kontrollstab 10 als Vorderansicht. Als CT-Bild sind auch die Wicklungsabschnitte der Rückseite sichtbar.

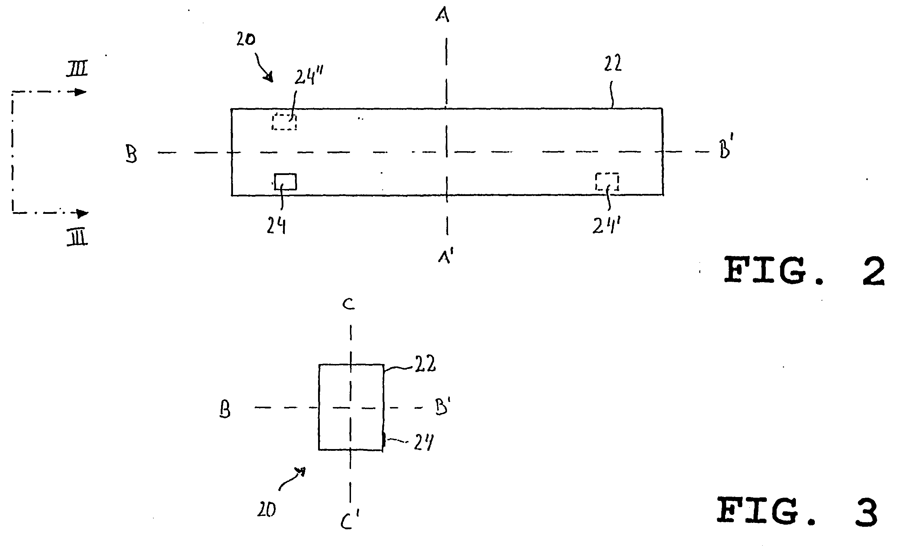

Die Figuren 2 und 3 zeigen als Ausführungsbeispiel der Erfindung einen zweiten Kontrollstab 20. Der zweite Kontrollstab 20 besteht aus einem Vierkantstab 22 mit Symmetrieebenen A ― A*'*, B ― B*'* und C ― C*'*. Auf dem Vierkantstab 22 ist eine Markierung 24 in Form eines Rechtecks aufgebracht. Die Markierung 24 ist bezüglich der Symmetrieebenen A ― A*'*, B ― B*'* und C ― C*'* asymmetrisch auf dem Vierkant aufgebracht und liegt außerhalb der drei Symmetrieebenen A - A*'*, B ― B*'* und C ― C*'*, so daß sie in einer seitenverkehrten Darstellung eine Positionsänderung erfährt. Abhängig von der Lage der Spiegelebene würde die Markierung 24 z.B. als Markierung 24*'* oder 24*''* auf der rechten Seite bzw. in der oberen linken Hälfte des Kontrollstabes 20 erscheinen, wie es in Figur 2 angedeutet ist.

Eine asymmetrisch angeordnete Markierung kann abweichend von der Rechteckform auch rund, oval oder andersartig geformt sein. Ebenso ist es möglich, einen Kontrollstab mit beliebigem Querschnitt zu verwenden, der vorzugsweise punktsymmetrisch ist.

## Patentansprüche

1. Kontrollkörper (10, 20) für ein bildgebendes Verfahren, bei dem ein Bild eines Objektes aufgrund unterschiedlicher physikalischer Eigenschaften von Teilbereichen erzeugt wird,
**gekennzeichnet** durch ein Material (14, 24) vorgegebener Dichte, das bezüglich der Symmetrieebenen (A-A*'*, B-B*'*, C-C*'*) des von dem Kontrollkörper (10, 20) umschlossenen Raumes asymmetrisch ausgebildet und/oder angeordnet ist.

2. Kontrollkörper (10) nach Anspruch 1, dadurch **gekennzeichnet**, daß der Kontrollkörper (10) eine freitragende schraubenförmige Spirale ist.

3. Kontrollkörper (10, 20) nach Anspruch 1, dadurch **gekennzeichnet**, daß eine asymmetrisch ausgebildete und/oder angeordnete Markierung (14, 24) in den Kontrollkörper (10, 20) eingebettet ist.

4. Kontrollkörper (10) nach Anspruch 1 oder 3, dadurch **gekennzeichnet**, daß der Kontrollkörper (10) ein Zylinderstab ist und die Markierung (14) ein Streifen auf der Mantelfläche mit zur Längsachse schrägem Verlauf ist.

5. Kontrollkörper (10) nach Anspruch 4, dadurch **gekennzeichnet**, daß die Markierung (14) wendelförmig gestaltet ist.

6. Kontrollkörper (10) nach Anspruch 4, dadurch **gekennzeichnet**, daß die Markierung (14) aus einem Band besteht.

7. Kontrollkörper (10) nach Anspruch 4, dadurch **gekennzeichnet**, daß die Markierung (14) aus einem Draht besteht.

8. Kontrollkörper (10) nach einem der Ansprüche 4 bis 7, **gekennzeichnet** durch einen mit Scheiben von Material unterschiedlicher Dichte gefüllten Innenraum.

9. Kontrollkörper (10) nach Anspruch 8, dadurch **gekennzeichnet,** daß sich die Markierung (14) im Innenraum des Kontrollkörpers befindet.
